# FASCICULE DE BREVET EUROPEEN

(11) **EP 3 058 983 B1**
(45) Date de publication et mention de la délivrance du brevet: **13.10.2021**
(21) Numéro de dépôt: 16154603.1
(22) Date de dépôt: 08.02.2016
(51) Int. Cl.: A61N 1/36, A61N 1/05

(54) **MICROSONDE DE DÉTECTION/STIMULATION, NOTAMMENT POUR LA NEUROMODULATION MULTIPOINT DU SYSTÈME NERVEUX CENTRAL**
MIKROSONDE ZUR DETEKTION/STIMULATION, INSBESONDERE FÜR DIE MEHRPUNKT-NEUROMODULATION DES ZENTRALEN NERVENSYSTEMS
DETECTION/STIMULATION MICROPROBE, IN PARTICULAR FOR MULTIPOINT NEUROMODULATION OF THE CENTRAL NERVOUS SYSTEM

(30) Priorité: 17.02.2015 FR 1551295
(43) Date de publication de la demande: 24.08.2016
(73) Titulaire: Sorin CRM SAS, 92140 Clamart Cedex (FR)
(72) Inventeur: SHAN, Nicolas, 91260 Juvisy sur Orge (FR); LAI, The-hoa, 95800 Courdimanche (FR)
(74) Mandataire: Grünecker Patent- und Rechtsanwälte PartG mbB

(56) Documents cités:
- EP-A1- 2 581 107
- EP-A1- 2 719 422
- CN-A- 104 274 902
- US-A1- 2006 089 697

## Description

L'invention concerne les "dispositifs médicaux implantables actifs" tels que définis par la directive 90/385/CEE du 20 juin 1990 du Conseil des communautés européennes.

Elle concerne plus précisément une microsonde de neuromodulation opérant par stimulation multipoint du système nerveux central.

Une telle sonde est typiquement destinée à être implantée dans le réseau veineux cérébral de manière à atteindre des zones cibles spécifiques du cerveau afin d'y appliquer des impulsions électriques de neurostimulation pour traiter certaines pathologies telles que la maladie de Parkinson, l'épilepsie, etc., techniques regroupées sous la dénomination générale DBS (*Deep Brain Stimulation,* stimulation profonde du cerveau). Il peut également s'agir de stimuler la moelle épinière, notamment pour le traitement de la douleur, ces techniques étant connues sous la dénomination générale SCS (*Spinal Cord Stimulation,* stimulation de la moelle épinière).

Ces techniques se distinguent par de nombreux aspects, notamment par les moyens utilisés, de celles qui sont employées en cardiologie ou pour d'autres types de stimulation nerveuse où c'est le système nerveux périphérique qui est stimulé, comme dans les techniques dites VNS (*Vagus Nerve Stimulation,* stimulation du nerf vague) ou analogues, où les électrodes sont placées au niveau des nerfs ou des muscles, donc dans des zones beaucoup plus aisément accessibles.

La spécificité des sondes pour la stimulation du système nerveux central résulte principalement dans le diamètre de ces sondes, impérativement inférieur à 1,5 French, soit 0,5 mm (d'où le terme de "microsonde"), ainsi que dans le nombre d'électrodes nécessaires pour permettre une stimulation "multipoint".

Le but de la présente invention est de proposer une structure de microsonde qui permette d'atteindre des zones profondes du cerveau telles que les régions, potentiellement connues comme efficaces pour un traitement par neuromodulation, connues sous le nom de noyau subthalamique (STN) ou *globus pallidus* interne (GPI), et de stimuler très précisément des zones cibles situées dans ces régions.

Les solutions actuelles de neurostimulation profonde utilisent en général une approche fortement invasive, basée sur la perforation du crâne et la mise en place de la sonde par un guidage externe.

Il serait toutefois souhaitable de pouvoir disposer de moyens permettant une approche beaucoup moins invasive, par un abord veineux mettant en œuvre des techniques comparables à celles utilisées pour la microcathétérisation du cerveau, utilisées dans le cadre de la neuroradiologie interventionnelle. À condition de pouvoir disposer d'une structure de diamètre suffisamment faible et capable de naviguer au sein du réseau veineux et artériel du cerveau, ces techniques pourraient être utilisées pour la pose d'une microsonde. La microsonde doit toutefois rester adaptée à une implantation permanente dans le cerveau.

L'utilisation dans ce contexte de microsondes connues se heurte toutefois à plusieurs difficultés majeures.

En premier lieu, des sondes de diamètre trop élevé peuvent causer des dégâts neurologiques importants lors de l'intervention chirurgicale d'implantation. Il est donc nécessaire de réduire très fortement le diamètre de la microsonde, mais en lui conservant toutefois d'excellentes propriétés de navigabilité au sein du réseau veineux pour permettre sa mise en place. Le réseau veineux artériel cérébral comprend en effet de fortes tortuosités et de nombreux embranchements, et il est indispensable d'éviter des traumatismes que pourrait engendrer une sonde trop rigide. Mais, inversement, une microsonde trop souple serait difficile à mettre en place, du fait d'une trop faible raideur en torsion pour permettre la transmission sur toute la longueur du corps de sonde, jusqu'à l'extrémité distale, d'un mouvement de rotation imprimé depuis l'extrémité proximale (manque de "torquabilité"). De plus, une microsonde trop souple ne pourrait pas progresser dans le réseau biologique sans arc-boutement sous l'effet d'une poussée axiale (manque de "pushabilité").

En deuxième lieu, il est souhaitable que la sonde implantable soit compatible avec les cathéters de 1,6 French (0,53 mm) tels que ceux déjà utilisés aujourd'hui en neuroradiologie interventionnelle par exemple pour la libération de dispositifs tels que des ressorts (*coils*) lors du traitement des anévrysmes intracrâniens. Ceci implique impérativement pour la sonde un diamètre hors-tout inférieur à 1,5 French (0,5 mm).

En troisième lieu, les électrodes d'une microsonde de neurostimulation doivent présenter une surface extrêmement réduite, de manière à pouvoir stimuler précisément les zones cibles sans risquer de produire des effets secondaires psychiatriques graves, ce qui survient malheureusement aujourd'hui dans un pourcentage important des interventions.

Enfin, en quatrième lieu, il est nécessaire de disposer sur une même microsonde d'un nombre très élevé d'électrodes de neurostimulation, toutes sélectionnables indépendamment, de manière à affiner au maximum la précision des points de contact stimulés, l'idéal étant de disposer d'au moins 8, de préférence de 20 à 100 électrodes programmables indépendamment, au surplus avec possibilité de sélectionner des électrodes situées dans des directions angulaires différentes sur une même position longitudinale de la sonde. Cette multiplication du nombre d'électrodes, et par voie de conséquence de conducteurs indépendants, ne doit bien entendu pas se faire au détriment du faible diamètre de la microsonde, indispensable pour réduire la traumaticité de celle-ci et offrir des possibilités d'abord vers des zones profondes du cerveau.

Diverses structures de sondes de neuromodulation à conducteurs multiples ont été proposées, par exemple dans les WO 2007/115198 A2, US 2006/0111768 A1 ou US 2010/0057176 A1, mais pour un nombre relativement réduit de conducteurs, donc d'électrodes programmables (de l'ordre d'une dizaine tout au plus).

Le US 2006/0089697 A1 décrit une sonde comprenant une pluralité de conducteurs bobinés indépendants, répartis autour d'un tube creux, l'ensemble étant protégé par un chemisage isolant externe. Le tube est traversé de part en part par une lumière centrale destinée à permettre l'insertion d'un mandrin de pose inséré dans cette lumière pendant l'implantation. Le diamètre hors-tout de cette structure (au moins 0,8 mm) est toutefois beaucoup trop important pour permettre d'atteindre les zonescibles les plus profondes du cerveau.

Le US 2013/0018445 A1 décrit une sonde de neurostimulation pouvant comporter jusqu'à 49 brins conducteurs enroulés en spirale et individuellement isolés, mais dans une application de stimulation d'un nerf périphérique situé dans un tissu musculaire ou adipeux, donc dans un environnement où ne se posent pas les contraintes de très faible diamètre et de navigabilité.

Les EP 2 581 107 A1 et EP 2 719 422 A1 (Sorin CRM) décrivent des structures de microsondes implantables dans des réseaux veineux, artériels ou lymphatiques. Ces microsondes sont toutefois essentiellement conçues pour une implantation dans le réseau coronaire veineux pour la stimulation du ventricule gauche du myocarde, donc dans des applications cardiologiques. Leur structure est en effet spécifiquement conçue pour résister aux contraintes de fatigue très sévères liées aux battements cardiaques, qui engendrent une fatigue du matériau sous l'effet des flexions répétées sur des centaines de millions de cycles, pouvant entrainer sa rupture et limiter sa durée de vie.

Ces aspects sont beaucoup moins critiques dans le cas d'une microsonde de neuromodulation DBS ou SCS, qui est implantée dans un milieu bien plus statique que le cœur et beaucoup moins sujet aux contraintes de fatigue. Bien plus, l'exigence majeure de multiplication du nombre d'électrodes indépendantes (typiquement au moins 8, de préférence 20 à 100 électrodes) ne peut pas être satisfaite par les structures de microsondes décrites dans ces documents, qui ne peuvent intégrer tout au plus que sept conducteurs indépendants dans le diamètre prescrit de 1,5 French (0,5 mm).

Ainsi, la présente invention a pour objet de résoudre le problème consistant à disposer d'une microsonde spécifiquement adaptée à la stimulation multipoint du système nerveux central, qui offre :
- la possibilité de multiplier le nombre de conducteurs dans une structure torsadée à la fois compacte et résistante à des sollicitations mécaniques en flexion et souple, en ayant jusqu'à 100 fils isolés dans une dimension inférieure à 0,5 mm ;
- la possibilité de réaliser, dans cette structure, des électrodes de très petite taille, et orientées dans plusieurs directions axiales ; et
- qui soit adaptée à une implantation sur le long terme dans les applications de stimulation neurologique permanente, après mise en place dans le réseau veineux cérébral.

À cet effet, l'invention propose une microsonde du type général divulgué par exemple par le EP 2 719 422 A1 précité, c'est-à-dire une microsonde multipolaire de diamètre hors-tout inférieur à 1,5 French (0,5 mm) comprenant une pluralité d'au moins huit fils conducteurs individuellement isolés et torsadés ensemble, chaque fil conducteur comprenant : un microcâble de cœur électriquement conducteur, apte à être relié en partie proximale à un pôle d'un générateur d'un dispositif médical implantable actif ; et une couche d'isolement entourant le câble de cœur, et présentant au moins une zone dénudée ménagée dans l'épaisseur de la couche d'isolement en partie distale pour former une électrode de détection/stimulation de la microsonde.

De façon caractéristique de l'invention, la microsonde comprend en outre une structure support centrale en forme de surface de révolution, cette structure support centrale étant dépourvue i) de fil conducteur et ii) de lumière centrale, et les fils conducteurs de ladite pluralité sont configurés en une couche d'un enroulement torsadé de fils conducteurs périphériques portés par la structure support centrale et circonférentiellement répartis sur celle-ci.

Selon diverses caractéristiques subsidiaires avantageuses :
- une partie des fils conducteurs périphériques sont configurés en une première couche directement portée par la structure support centrale et une autre partie des fils conducteurs périphériques sont configurés en une deuxième couche portée par ladite première couche ;
- la structure support centrale comprend un élément cylindrique homogène unique de section pleine ou tubulaire, ou bien une pluralité d'éléments cylindriques homogènes de section pleine ou tubulaire toronnés ensemble ;
- la structure support centrale comprend un bobinage d'un circuit de protection contre les surintensités induites en situation d'examen IRM ;
- le diamètre hors-tout de la structure support centrale est supérieur au diamètre unitaire d'un fil conducteur individuel ;
- à l'encontre des sollicitations en flexion, la structure support centrale présente une aptitude à la déformation élastique supérieure à celle de l'ensemble des fils conducteurs individuels ;
- la structure support centrale est une structure effilée avec un diamètre décroissant de la région proximale vers la région distale, notamment une structure comprenant une partie conique de transition entre une partie proximale cylindrique de diamètre nominal supérieur au diamètre unitaire d'un fil conducteur individuel, et une partie distale cylindrique de diamètre inférieur à celui de la partie proximale ;
- la pluralité de fils conducteurs comprend de 10 à 50 fils conducteurs par couche ;
- le diamètre unitaire d'un fil conducteur individuel est compris entre 15 et 25 µm.

On va maintenant décrire un exemple de mise en œuvre de la présente invention, en référence aux dessins annexés où les mêmes références désignent d'une figure à l'autre des éléments identiques ou fonctionnellement semblables.
La Figure 1 illustre de façon générale un exemple d'implantation d'une microsonde selon l'invention dans le réseau vasculaire cérébral.
Les Figures 2a et 2b montrent, respectivement en section droite et en vue latérale, la structure d'ensemble de la microsonde selon l'invention.
Les Figures 3a à 3d illustrent diverses variantes de la structure support centrale de la microsonde selon l'invention.
La Figure 4 illustre une mise en œuvre particulière, avec une structure support centrale comportant une portion conique.
La Figure 5 illustre, en section droite, un exemple de réalisation d'une microsonde selon l'invention à vingt-six électrodes.
Les Figures 6 et 7 illustrent, respectivement en vue de côté et en section droite, un mode de réalisation de la microsonde selon l'invention comportant deux couches de conducteurs périphériques superposées.
La Figure 8 est un organigramme décrivant les différentes phases de la procédure d'implantation d'une microsonde selon l'invention.

On va maintenant décrire un exemple de réalisation de l'invention.

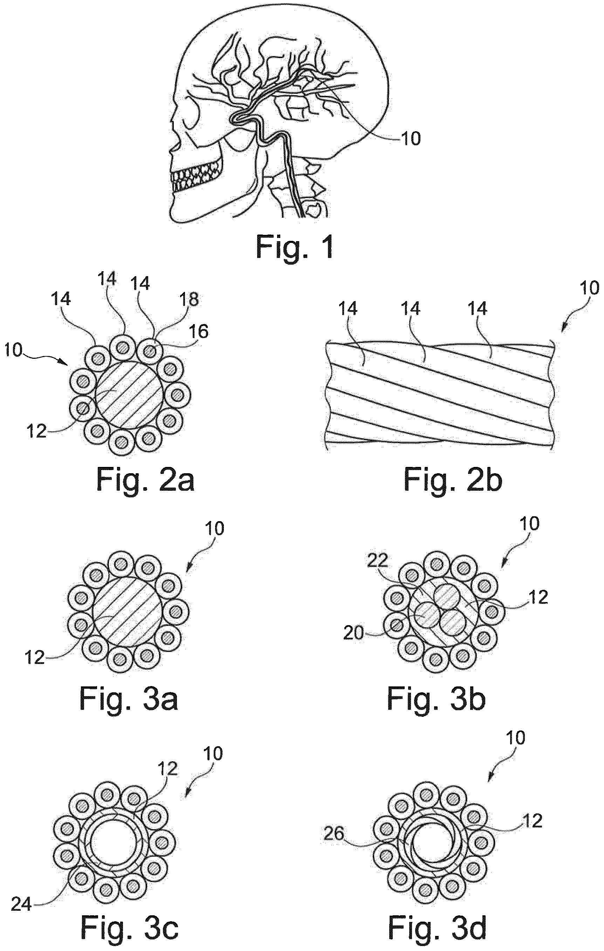

Sur la Figure 1, on a illustré de façon générale une microsonde 10 selon l'invention, implantée dans le réseau vasculaire cérébral afin de pouvoir stimuler sélectivement des zones profondes du cerveau par application localisée d'impulsions électriques. Les électrodes de cette microsonde peuvent par ailleurs faire fonction, le cas échéant, d'électrodes de détection pour recueillir des potentiels électriques produits localement.

La stimulation de zones cibles du cerveau permet en particulier de mettre en œuvre des techniques de neuromodulation destinées à traiter des pathologies telles que la maladie de Parkinson, l'épilepsie et autres maladies neurologiques.

Il est de ce fait nécessaire d'accéder à des régions profondes du cerveau, difficilement atteignables aujourd'hui avec les technologies connues.

Les microsondes de stimulation envisageables à cet effet doivent présenter non seulement une grande robustesse, afin de garantir la biostabilité à long terme (ces microsondes sont destinées à être implantées de façon permanente), mais également une taille très réduite, avec un diamètre hors-tout inférieur à 1,5 French (0,5 mm). En particulier, des microsondes de 1,5 French seraient avantageusement compatibles avec des cathéters de 1,6 French (0,53 mm), déjà utilisés aujourd'hui en neuroradiologie interventionnelle par exemple pour la libération de dispositifs tels que des ressorts *(coils)* lors du traitement des anévrysmes intracrâniens.

Par ailleurs, ces microsondes doivent posséder un nombre élevé d'électrodes, typiquement 20 à 100 électrodes, sélectionnables indépendamment de manière à pouvoir choisir très précisément les zones de stimulation en fonction de l'effet recherché. Il est également souhaitable de pouvoir choisir la direction axiale dans laquelle agissent ces électrodes, afin d'optimiser l'effet produit et éviter l'apparition d'effets secondaires indésirables.

Les Figures 2a et 2b montrent, respectivement en section droite et en vue de côté, la structure de microsonde proposée par la présente invention.

La microsonde 10 comprend une structure support centrale 12 en forme de surface de révolution, couverte à sa périphérie d'une pluralité de fils conducteurs périphériques 14 portés par cette structure support centrale 12 et circonférentiellement répartis sur celle-ci.

Chacun des fils conducteurs périphériques 14 comprend un microcâble de cœur 16 électriquement conducteur et une couche d'isolement 18 entourant le câble de cœur.

Le microcâble de cœur peut être réalisé en un métal conducteur tel qu'un alliage platine-iridium, un acier MP35N, du nitinol, etc. Diverses structures de câble de cœur appropriées à cette application sont décrites en particulier dans le EP 2 581 107 A1 précité (Sorin CRM), auquel on pourra se référer pour plus de détails. Il est également possible d'utiliser pour le microcâble de cœur 16 des matériaux tels que des nanotubes de carbone, qui sont des matériaux de choix pour leurs caractéristiques de résistance mécanique exceptionnelles et leurs très bonnes propriétés de conductivité électrique.

Pour la couche d'isolement 18, on pourra utiliser des matériaux tels que les polyuréthannes (PU), polyesters (PET), polyamides (PA), polycarbonates (PC), polyimides, polymères fluorés, le polyéther-éther-cétone (PEEK), le poly-p-xylylène (parylène), ou le polyméthacrylate de méthyle (PMM). Cependant, on privilégiera les matériaux à forte inertie chimique comme les polymères fluorés, qui présentent également une très bonne qualité d'isolation, notamment le PTFE (polytétrafluoroéthylène), le FEP (propylène perfluoré), le PFA (résine de copolymère perfluoroalkoxy), le THV (tétrafluoroéthylène, hexafluoropropylène, fluorure de vinylidène), le PVDF (polyfluorure de vinylidène), l'EFEP (éthylène propylène éthylène fluoré), ou l'ETFE (éthylène tétrafluoroéthylène).

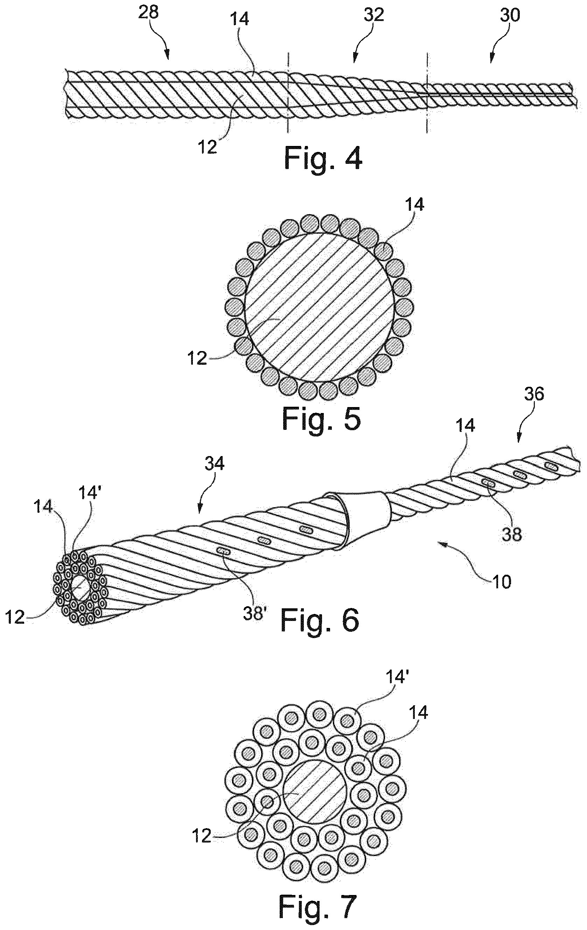

Chacun des fils conducteurs présente dans la région distale de la sonde au moins une zone dénudée (comme illustré en 38 ou 38' sur la Figure 6) ménagée dans l'épaisseur de la couche d'isolement pour former une électrode de détection/stimulation de la microsonde.

L'architecture de la microsonde selon l'invention, avec un enroulement torsadé de fils conducteurs périphériques isolés 14 portés par une structure support centrale 12, permet de réduire la taille de la sonde dans des proportions très importantes tout en garantissant un nombre important de lignes électriques isolées, reliées à des électrodes indépendantes et donc programmables selon des configurations multiples par le générateur auquel sont raccordées les microsondes.

De préférence, pour minimiser sa taille, cette structure ne comporte pas de lumière centrale (c'est-à-dire de canal débouchant aux deux extrémités de la sonde), de sorte que pour la pose de la microsonde le guidage se fera par l'extérieur via un cathéter de pose, et non par un fil-guide inséré dans une lumière centrale.

Les Figures 3a à 3d illustrent diverses variantes de réalisation de la structure support centrale 12 :
- Figure 3a : une âme simple, formée d'un noyau monobrin plein homogène ;
- Figure 3b : une âme constituée d'un noyau multibrin, avec plusieurs brins 20 noyés dans un revêtement 22 ;
- Figure 3c : une âme tubulaire 24 ;
- Figure 3d : une structure support incorporant dans son âme un bobinage 26 d'un circuit de protection contre les surintensités induites en situation d'examen IRM.

Les matériaux de la structure support centrale 12 sont choisis et/ou combinés en fonction des propriétés finales recherchées pour la microsonde, de manière à procurer à cette dernière des fonctionnalités multiples telles que :
- radio-opacité, par incorporation dans le matériau de la structure support centrale 12 d'un métal tel que le tantale, le palladium, l'or ou un alliage platine-iridium ;
- mémoire de forme, par emploi de polymères présentant des propriétés de flexibilité et de hautes performances élastiques tels que PEEK, PA, PEBA, PU, PET ou PFE ;
- flexibilité, "pushabilité" et "torquabilité" : la structure support centrale 12 doit en particulier présenter, à l'encontre des sollicitations en flexion, une aptitude à la déformation élastique qui soit supérieure à celle des fils conducteurs individuels 14, cette aptitude à la déformation en flexion permettant d'aller dans le réseau cérébral profond.

Comme illustré Figure 4, pour améliorer encore les performances de la microsonde au moment de son implantation, notamment l'aptitude à la faire progresser dans le sens de la longueur sans arc-boutement, il est possible de prévoir une partie proximale cylindrique 28 de diamètre nominal, reliée à une partie distale 30 de diamètre plus réduit par l'intermédiaire d'une partie conique de transition 32. La partie proximale 28 de diamètre supérieur procure de la "pushabilité", c'est-à-dire l'aptitude à faire progresser la microsonde sous l'effet d'une sollicitation axiale appliquée par exemple au moyen d'une poignée de manoeuvre depuis l'extrémité proximale, tandis que la partie distale 30 beaucoup plus fine permet d'atteindre aisément des vaisseaux profonds, étroits, de la région cérébrale.

La Figure 5 illustre un exemple de réalisation d'une microsonde selon l'invention à vingt-six électrodes, comprenant donc vingt-six fils conducteurs périphériques 14 portés par une structure support centrale 12.

La très grande compacité de la structure permet d'utiliser des fils isolés pouvant présenter un diamètre aussi faible que 15 à 25 µm, de sorte qu'il est possible de placer typiquement jusqu'à cinquante fils conducteurs, et donc de disposer d'autant d'électrodes indépendantes, dans un diamètre hors-tout de 0,40 mm pour un diamètre de fil conducteur unitaire de 25 µm (le nombre de fils conducteurs augmentant géométriquement en réduisant la taille de ces conducteurs).

Les Figures 6 et 7 illustrent une variante comprenant deux couches superposées de conducteurs sur la structure support centrale, avec une première couche de fils conducteurs périphériques 14 directement portés par la structure support centrale 12, et une seconde couche de fils conducteurs périphériques 14' portés par la première couche de conducteurs 14. Il est possible d'exploiter indépendamment toutes les couches de la structure et multiplier ainsi les possibilités, avec jusqu'à plus de cent conducteurs 14 ou 14' exploitables indépendamment dans une même structure dont le diamètre hors tout ne dépasse pas 0,5 mm. Les deux couches de fils conducteurs respectifs 14 et 14' peuvent être décalées axialement, avec une zone proximale 34 où c'est la seconde couche des conducteurs 14' qui est apparente, et une zone distale 36 où c'est la surface des conducteurs 14 de la première couche qui est apparente. La zone proximale 34 sera celle portant les électrodes 38' reliées aux conducteurs 14', tandis que la zone distale 36 sera celle portant les électrodes 38 reliées aux conducteurs 14.

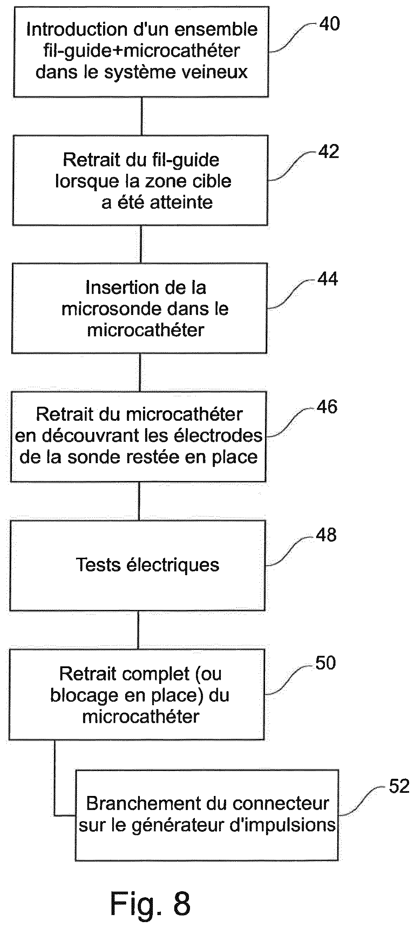

La Figure 8 illustre de façon schématique les différentes phases de la procédure d'implantation de la sonde que l'on vient de décrire.

Cette procédure est semblable à celle d'une sonde conventionnelle, mis à part le fait qu'en raison de l'absence de lumière interne il n'est pas possible d'utiliser un fil-guide pour mettre en place la sonde et la guider dans les vaisseaux du réseau cérébral. Il sera alors nécessaire d'utiliser à la place un microcathéter, selon des procédures en elles-mêmes connues des praticiens.

La première étape (bloc 40) consiste à introduire dans le système veineux jusqu'à la zone cible un ensemble formé d'un microcathéter et d'un fil-guide.

Lorsque cette zone cible est atteinte (bloc 42) le fil-guide est retiré, en laissant en place le microcathéter.

La microsonde est ensuite introduite dans le microcathéter (bloc 44), puis le microcathéter est partiellement retiré pour découvrir progressivement les électrodes de la microsonde (bloc 46).

Les tests électriques sont alors effectués, de façon automatique ou manuelle (bloc 48). Une fois ces tests effectués, lorsque l'on est sûr que la sonde est parfaitement fonctionnelle, le microcathéter est complètement retiré (bloc 50), ou bien verrouillé en place s'il s'agit d'un microcathéter pouvant être implanté à demeure tel que celui décrit par exemple dans le EP 2 682 151 A1 (Sorin CRM).

Le connecteur de la sonde peut être alors relié au générateur d'impulsions (bloc 50) afin que celui-ci puisse délivrer au cerveau les impulsions de neurostimulation.

## Revendications

1. Une microsonde de détection/stimulation multipolaire de diamètre hors-tout inférieur à 1,5 French (0,5 mm), destinée à être implantée dans le réseau veineux cérébral profond, comprenant une pluralité d'au moins huit fils conducteurs (14) individuellement isolés et torsadés ensemble, chaque fil conducteur comprenant :
- un microcâble de cœur (16) électriquement conducteur, apte à être relié en partie proximale à un pôle d'un générateur d'un dispositif médical implantable actif ; et
- une couche d'isolement (18) entourant le câble de cœur, et présentant au moins une zone dénudée (38) ménagée dans l'épaisseur de la couche d'isolement en partie distale pour former une électrode de détection/stimulation de la microsonde,
la microsonde comprenant en outre une structure support centrale (12) en forme de surface de révolution, cette structure support centrale étant dépourvue i) de fil conducteur et ii) de lumière centrale,
et dans laquelle les fils conducteurs (14) de ladite pluralité sont configurés en une couche d'un enroulement torsadé de fils conducteurs périphériques (14) portés par la structure support centrale et circonférentiellement répartis sur celle-ci.

2. La microsonde de la revendication 1, dans laquelle une partie desdits fils conducteurs périphériques (14) sont configurés en une première couche directement portée par la structure support centrale (12) et une autre partie desdits fils conducteurs périphériques (14') sont configurés en une deuxième couche portée par ladite première couche.

3. La microsonde de la revendication 1, dans laquelle la structure support centrale (12) comprend un élément cylindrique homogène unique de section pleine ou tubulaire.

4. La microsonde de la revendication 1, dans laquelle la structure support centrale (12) comprend une pluralité d'éléments cylindriques homogènes (20) de section pleine ou tubulaire toronnés ensemble.

5. La microsonde de la revendication 1, dans laquelle la structure support centrale (12) comprend un bobinage (26) d'un circuit de protection contre les surintensités induites en situation d'examen IRM.

6. La microsonde de la revendication 1, dans laquelle le diamètre hors-tout de la structure support centrale (12) est supérieur au diamètre unitaire d'un fil conducteur individuel (14).

7. La microsonde de la revendication 1, dans laquelle, à l'encontre des sollicitations en flexion, la structure support centrale (12) présente une aptitude à la déformation élastique supérieure à celle de l'ensemble des fils conducteurs individuels (14).

8. La microsonde de la revendication 1, dans laquelle la structure support centrale (12) est une structure effilée avec un diamètre décroissant de la région proximale (28) vers la région distale (30).

9. La microsonde de la revendication 8, dans laquelle la structure support centrale (12) comprend une partie conique de transition (32) entre une partie proximale cylindrique (28) de diamètre nominal supérieur au diamètre unitaire d'un fil conducteur individuel, et une partie distale cylindrique (30) de diamètre inférieur à celui de la partie proximale.

10. La microsonde de la revendication 1, dans laquelle ladite pluralité de fils conducteurs (14) comprend de 10 à 50 fils conducteurs par couche.

11. La microsonde de la revendication 1, dans laquelle le diamètre unitaire d'un fil conducteur individuel (14) est compris entre 15 et 25 µm.

## Patentansprüche

1. Multipolare Mikrosonde zur Detektions-/Stimulations mit einem Gesamtdurchmesser von weniger als 1,5 French (0,5 mm) zur Implantation in das tiefe zerebrale Venennetz, umfassend eine Vielzahl von mindestens acht einzeln isolierten, miteinander verdrillten Leitungsdrähten (14), wobei jeder Leitungsdraht Folgendes umfasst:
- ein elektrisch leitendes Mikrokernkabel (16), das proximal mit einem Pol eines Generators einer aktiven implantierbaren medizinischen Vorrichtung verbunden werden kann; und
- eine Isolierschicht (18), die das Kernkabel umgibt und mindestens einen freiliegenden Bereich (38) aufweist, der in der Dicke der Isolierschicht distal ausgebildet ist, um eine Detektions-/Stimulationselektrode der Mikrosonde zu bilden,
die Mikrosonde umfasst ferner eine zentrale Trägerstruktur (12) in Form einer Rotationsfläche, wobei die zentrale Trägerstruktur frei ist von i) Leitungsdraht und ii) zentralem Licht,
und wobei die Vielzahl von Leiterdrähten (14) als eine Schicht von verdrillten Wicklungen von peripheren Leiterdrähten (14) konfiguriert ist, die von der zentralen Trägerstruktur getragen werden und in Umfangsrichtung darüber verteilt sind.

2. Mikrosonde nach Anspruch 1, wobei ein Teil der peripheren Zuleitungen (14) als eine erste Schicht konfiguriert ist, die direkt von der zentralen Trägerstruktur (12) getragen wird, und ein anderer Teil der peripheren Zuleitungen (14') als eine zweite Schicht konfiguriert ist, die von der ersten Schicht getragen wird.

3. Mikrosonde nach Anspruch 1, wobei die zentrale Trägerstruktur (12) ein einziges homogenes zylindrisches Element mit massivem oder rohrförmigem Querschnitt umfasst.

4. Mikrosonde nach Anspruch 1, wobei die zentrale Trägerstruktur (12) eine Vielzahl homogener zylindrischer Elemente (20) mit massivem oder rohrförmigem Querschnitt umfasst, die miteinander verseilt sind.

5. Mikrosonde nach Anspruch 1, wobei die zentrale Trägerstruktur (12) eine Wicklung (26) einer Schutzschaltung gegen induzierte Überströme in einer MRT-Untersuchungssituation umfasst.

6. Mikrosonde nach Anspruch 1, wobei der Gesamtdurchmesser der zentralen Trägerstruktur (12) größer ist als der Einheitsdurchmesser eines einzelnen Leitungsdrahtes (14).

7. Mikrosonde nach Anspruch 1, wobei die zentrale Trägerstruktur (12) bei Biegebeanspruchung ein höheres elastisches Verformungsvermögen aufweist als alle Einzeldrähte (14).

8. Mikrosonde nach Anspruch 1, wobei die zentrale Stützstruktur (12) eine sich verjüngende Struktur mit abnehmendem Durchmesser vom proximalen Bereich (28) zum distalen Bereich (30) ist.

9. Mikrosonde nach Anspruch 8, wobei die zentrale Stützstruktur (12) einen sich verjüngenden Übergangsbereich (32) zwischen einem zylindrischen proximalen Bereich (28) mit einem Nenndurchmesser, der größer ist als der Einheitsdurchmesser eines einzelnen Leitungsdrahtes, und einem zylindrischen distalen Bereich (30) mit einem kleineren Durchmesser als der proximale Bereich aufweist.

10. Mikrosonde nach Anspruch 1, wobei die Vielzahl der leitenden Drähte (14) 10 bis 50 leitende Drähte pro Schicht umfasst.

11. Mikrosonde nach Anspruch 1, wobei der Einheitsdurchmesser eines einzelnen Leitungsdrahtes (14) zwischen 15 und 25 µm liegt.

## Claims

1. A multipolar detection/stimulation microprobe of less than 1.5 French (0.5 mm) overall diameter, for implantation in the deep cerebral venous network, comprising a plurality of at least eight individually insulated lead wires (14) twisted together, each lead wire comprising:
- an electrically conductive micro core cable (16) adapted to be connected proximally to a pole of a generator of an active implantable medical device; and
- an insulation layer (18) surrounding the core cable, and having at least one exposed area (38) formed in the thickness of the insulation layer distally to form a detection/stimulation electrode of the microprobe,
the microprobe further comprising a central support structure (12) in the form of a surface of revolution, said central support structure being free of i) conducting wire and ii) central light,
and wherein said plurality of conductor wires (14) are configured as a layer of twisted winding of peripheral conductor wires (14) carried by and circumferentially distributed over the central support structure.

2. The microprobe of claim 1, wherein a portion of said peripheral leads (14) are configured as a first layer directly carried by the central support structure (12) and another portion of said peripheral leads (14'⁾ are configured as a second layer carried by said first layer.

3. The microprobe of claim 1, wherein the central support structure (12) comprises a single homogeneous cylindrical member of solid or tubular section.

4. The microprobe of claim 1, wherein the central support structure (12) comprises a plurality of homogeneous cylindrical elements (20) of solid or tubular cross-section stranded together.

5. The microprobe of claim 1, wherein the central support structure (12) comprises a winding (26) of a protection circuit against induced overcurrents in an MRI examination situation.

6. The microprobe of claim 1, wherein the overall diameter of the central support structure (12) is greater than the unit diameter of an individual lead wire (14).

7. The microprobe according to claim 1, wherein the central support structure (12) has a higher elastic deformation capacity than all the individual wires (14) when subjected to bending stresses.

8. The microprobe of claim 1, wherein the central support structure (12) is a tapered structure with decreasing diameter from the proximal region (28) to the distal region (30).

9. The microprobe of claim 8, wherein the central support structure (12) comprises a tapered transition portion (32) between a cylindrical proximal portion (28) of nominal diameter greater than the unit diameter of an individual lead wire, and a cylindrical distal portion (30) of smaller diameter than the proximal portion.

10. The microprobe of claim 1, wherein said plurality of conductive wires (14) comprises from 10 to 50 conductive wires per layer.

11. The microprobe of claim 1, wherein the unit diameter of an individual lead wire (14) is between 15 and 25 µm.
